# EUROPEAN PATENT SPECIFICATION

(11) **EP 1 261 344 B1**
(45) Date of publication and mention of the grant of the patent: **16.06.2004**
(21) Application number: 01908522.4
(22) Date of filing: 16.02.2001
(51) Int. Cl.: A61K 31/519, A61P 25/28

(54) **USE OF INDOLOQUINOXALINES FOR THE TREATMENT OF MULTIPLE SKLEROSIS**
VERWENDUNG VON INDOLOQUINOXALINEN ZUR BEHANDLUNG DER MULTIPLEN SKLEROSE
UTILISATION DES INDOLOQUINOXALINES POUR LE TRAITEMENT DE LA SCLEROSE EN PLAQUES

(30) Priority: 18.02.2000 SE 0000537
(43) Date of publication of application: 04.12.2002
(73) Proprietor: OxyPharma AB, 100 74 Stockholm (SE)
(72) Inventor: MÖLLER, Lennart, S-640 61 Stallarholmen (SE); BERGMAN, Jan, S-163 60 Sp nga (SE)
(74) Representative: Halldin, Bo
(86) International application number: PCT/SE2001/000326
(87) International publication number: WO 2001/060371

(56) References cited:
- No documents have been disclosed.

## Description

The present invention relates to the use of a compound of the general formula I 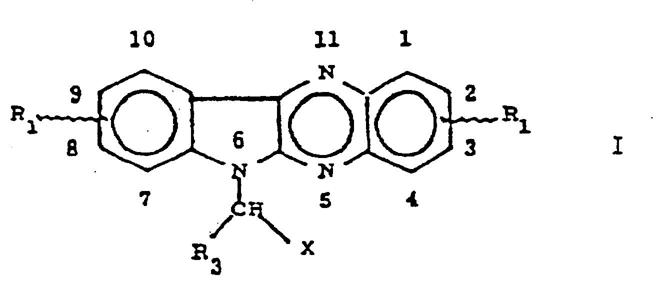 for preparing a drug for treatment of MS (multiple sclerosis).

MS is a chronic disease with its origin in the central nervous system (CNS) that often leads to severe consequences. MS can be mild with minor symptoms to severe paralysis and loss of vision. The diagnosis is most common between the ages of 20 to 40 and thereafter the disease continues the remaining life span. Sometimes MS develops rapidly, while in other cases the afflicted persons can live for many decades only with some disabilities.

MS is more frequent at northern latitudes. Depending on region in the western world the prevalence varies with 50-150 cases per 100,000. In the US only, some 250,000-350,000 have the MS diagnosis. Females have a double risk, compared to males, to develop MS.

It is generally accepted that the immune defense of a patient with MS attacks the CNS, while the exact mechanisms are unknown. Due to inflammation of the nerve isolation (myeline) there are dysfunctions and short-circuits of nerve fibers and thereby effects on the muscles controlled by these nerves.

The treatment of MS is focused on the reduction of symptoms. To cure or stop the MS disease is not possible with today's knowledge. Consequently there does not exist any drug to cure or delay onset of the disease. Treatments used are:
#Transplantation of bone marrow and treatment of cytostatics and lifelong administration of immunosupressive drugs. This method could work for some patients but it is very expensive and includes several risks for the patient. Administration of cytostatics is still considered to be controversial in treatment of MS since the effects are unclear and potential side- effects are severe.
#There are two drugs used with the aim to cure or delay the MS disease; Interferon-beta (trademarks Avonex^{R} and Betaseron^{R}) can reduce the symptoms among certain groups of patients and is therefore administered to most patients for ethical reasons. The effect is unclear for the population of MS patients and it is a very expensive treatment.

Glatiramer acetate (trademark Copaxone ^{R} ) can for some patients reduce the frequence of attacks, but the side effects are substantial and there is a problem to distinguish the symptoms of the MS disease and side effects.

Today there does not exist an effective treatment for MS. The treatment is focused on reducing symptoms. Tests with transplantation and different drug treatments to cure the disease have so far not shown any solutions. It can work for some patients, although there are risks, side effects and very high costs involved. MS is a rather common disease that appears early in life. In addition it is a life long disease with severe symptoms. The demand for drugs that can protect the MS patients for the severe development of the disease is therefore of high priority.

According to the present invention it has surprisingly been found that a substituted indolo-quinoxaline of the following general formula I can be used for preparing a drug for treatment of MS.

The compound which according to the present invention is to be used for preparing a drug for treatment of MS is a compound of the following general formula I 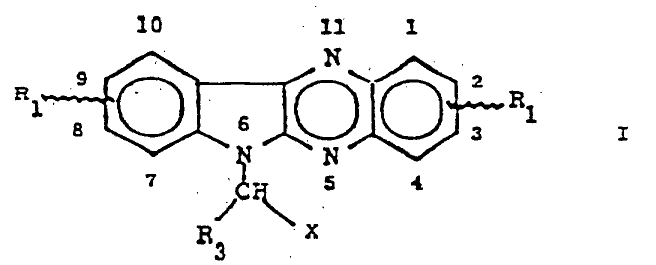 wherein R₁ represents hydrogen or one or several, preferably 1 to 4, similar or different substituents in the positions 1-4 and/or 7-10, selected from halogen, preferably Br, lower alkyl/alkoxy group having not more than 4 carbon atoms, trifluoromethyl group, trichloromethyl group; and in one of the positions 7-10 R₁ can be a hydroxyl group;
X is a group -(CH₂)ₙ-R₂, wherein R₂ represents a nitrogen containing basic residue such as NH₂, NHR₄ or NR₅R₆, wherein R₄, R₅ and R₆ independently are lower alkyl or cycloalkyl and n is an integer of from 1 to 4 and R₃ represents hydrogen; lower alkyl/cycloalkyl group having not more than 4 carbon atoms, and the physiologically acceptable addition. products of the compounds with acids and halogen adducts, preferably adducts with iodine, iodine monochloride or iodine monobromide.
R₁ is preferably selected from hydrogen and lower alkyl groups, especially methyl. More preferably R₁ is methyl in positions 2 and 3 and hydrogen in the other positions.
Suitable compounds are such compounds wherein R₁ is hydroxy in one of the positions 7-10, especially in position 9.

The compounds used according to the present invention and their preparation are described in EP patent 0238459 and US patent 4,990,510 which are incorporated herein by reference.

A compound which has proven to be especially effective is the compound of the following formula II

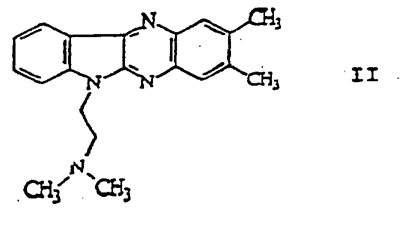

In the drawings:
**Figure 1** shows the results obtained in an EAE model test.
**Figure 2** shows the results obtained in an EAE rat model test with different doses of a compound used according to the present invention.
**Figure 3** shows the effect of pre-treatment with a compound used according to the present invention.
**Figure 4** shows the effect of after-treatment with a compound used according to the present invention.

The EAE (experimental autoimmune encephalomyelitis) model is a generally accepted animal model for the acute MS symptoms ((1)Ruuls et al, J. Immunology, 1996, 157, 5721-5731; (2)van der Medide et al, J. Neuroimmunology, 1998, 84, 14-23; (3)Smith et al, Nature Medicine,2000, 6, 1, 62-66). The model is based on Lewis rats that day 0 are induced by 20 µg myeline peptide (MBP 68-86) och 2 mg of Myobacterium tuberculosis. After one week severe CNS symptoms appear that are by double blind examination given a value - a clinical score. The higher value, the more severe effect. The scale is 0-5. After some 14 days there is a peak in symptoms followed by a decline back to the normal situation.

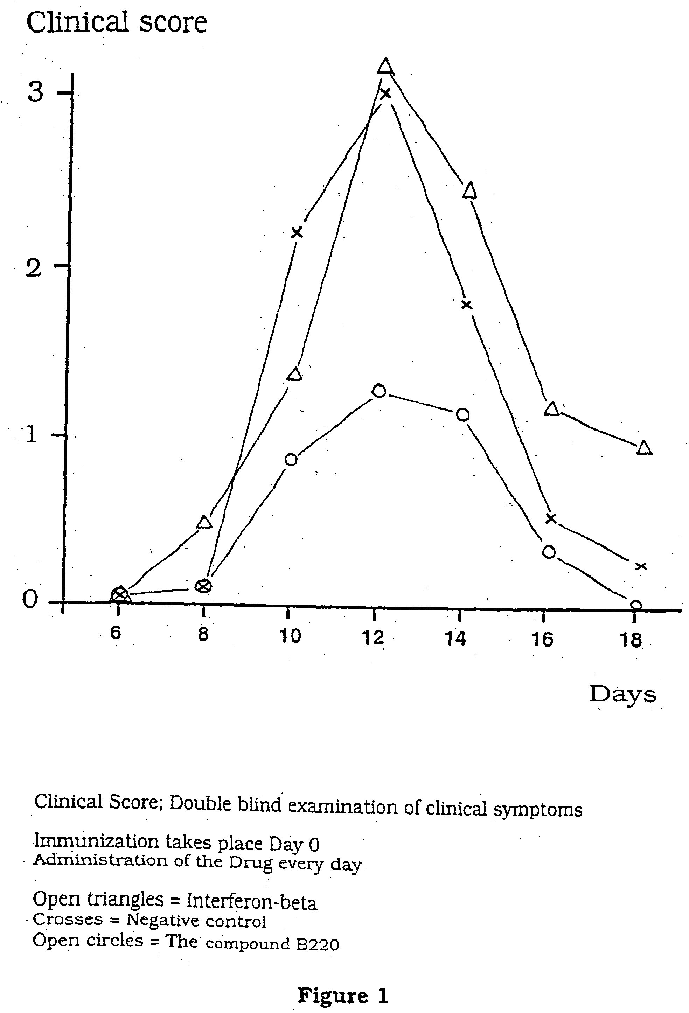

The negative control has no treatment except the immunization (day 0) to induce the acute MS response. In **Figure 1** the results from the negative control, Interferon-beta and a compound used according to the present invention, 2,3-dimethyl(dimethylaminoethyl)-5H-indolo-(2,3-b)quinoxaline, (B-220) are shown. From the figure it can be seen that Interferon-beta has no reducing effect of the CNS symptoms The lower curve represents the tested substance used according to the present invention, which substance surprisingly and unexpectedly reduces the CNS symptoms with 2/3.

In the test Interferon-beta was administered daily with 3 x 10⁵ U/animal which is a medium dose (2). The tested substance(B-220), was administered daily with 6 mg/animal, a dose that can be increased since the margin to toxic effects is wide and most likely further reduce the symptoms. It is to be noted that the acute toxicity of the tested substance used according to the present invention is low, which is exemplified with the following:
LD₅₀, oral, rat; >800 mg/kg bw
LD₅₀, intravenous, rat; >100 mg/kg bw
NOEL, intravenous, rat; 12.5 mg/kg bw
NOEL, cutaneous, rabbit; 200 mg/kg bw
(NOEL=No Observable Effect Level)

The chronic toxicity has been tested up to 270 days on mice and the substance has not induced toxicity, on the contrary the substance has protected the animals for different health effects.

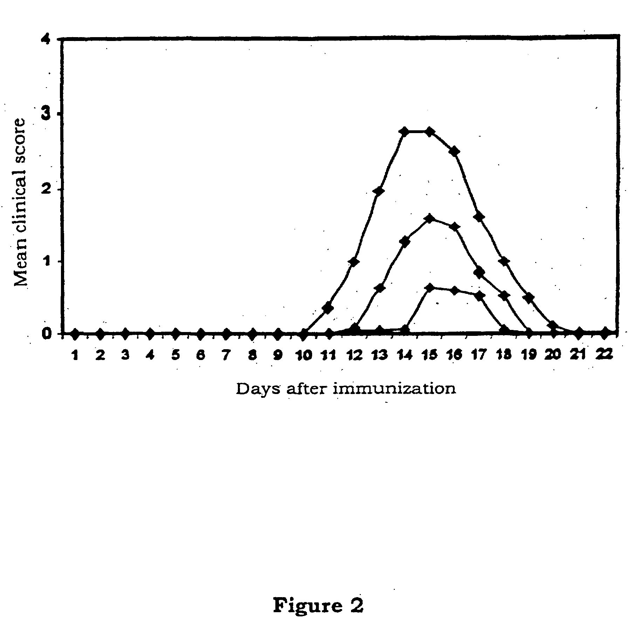

In the well established EAE rat model for multiple sclerosis (MS) a compound used according to the present invention, B-220, was shown to down-regulate the clinical symptoms (clinical score) in a dose dependent manner. The results are shown in **Figure 2**. At the highest dose, 12 mg/animal, (lower curve) the onset is delayed approximate 4 days, the recovery starts approximate 3 days earlier and the total effect is dramatically lowered. A majority of the animals do not show any symptoms at all in this group. Symptom grading 1 is thus a very weak and mild effect while grading 3 is a severe paralysis. The intermediate curve illustrates a dose of 6 mg/animal and the onset is delayed approximate 2 days, the recovery starts approximate 2 days earlier and the total effect is substantially lower as compared to the control, highest curve, where no B-220 is added.

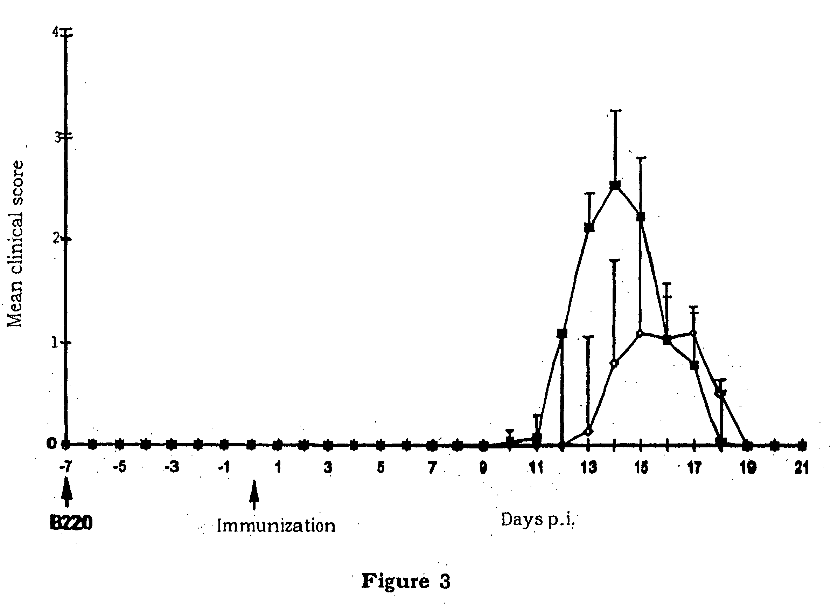

In **Figure 3** pre-treatment with B-220 before the onset of the disease is shown. The highest curve is illustrating the control without any added B-220 while the lower curve is after administration of B-220 of 6 mg/animal. As seen from the Figure the pre-treatment results in a clear lowered and delayed MS effect. Administration of B-220 was from -7 to +7 days in relation to the onset of disease (day 0).

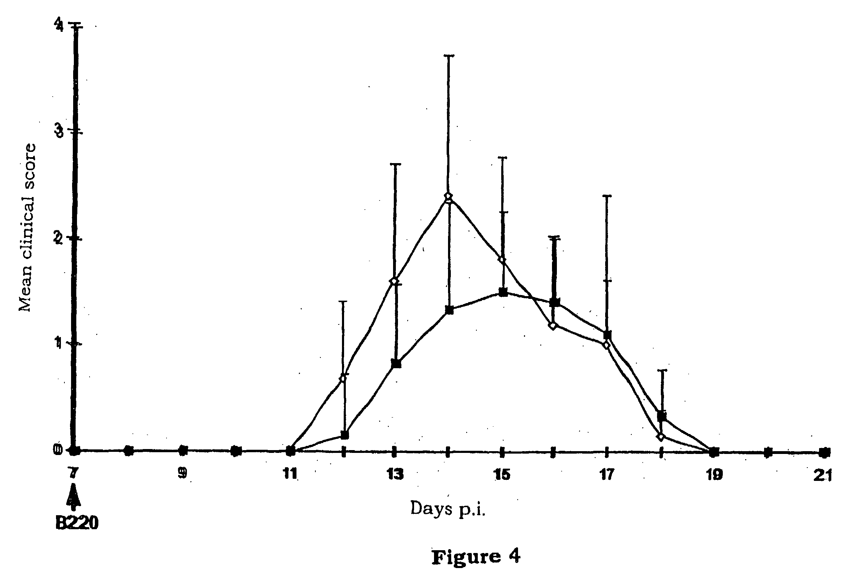

In **Figure 4** after-treatment with B-220 starting from day 7 from the time point when the disease was initiated (day 0) and throughout the experiment is shown wherein the highest curve is a control without any addition of B-220 and the lower curve is representing a dose of 6 mg/animal of B-220. As seen from the Figure the after-treatment with B-220 lowered the MS effect in the rats.

The vertical lines in both Figure 3 and Figure 4 represent mean±standard deviation.

A suitable dosage range for humans is 1 to 50 mg/kg body weight.

## Claims

1. Use of a compound which is a substituted indoloquinoxaline of the general formula I 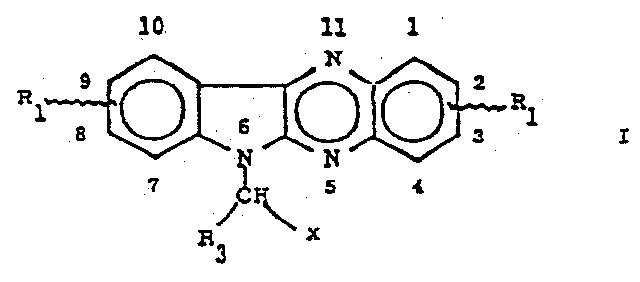 wherein R₁ represents hydrogen or one or several, preferably 1 to 4, similar or different substituents in the positions 1-4 and/or 7-10, selected from halogen, preferably Br, lower alkyl/alkoxy group having not more than 4 carbon atoms, trifluoromethyl group, trichloromethyl group; and in one of the positions 7-10 R₁ can be a hydroxyl group;
X is a group -(CH₂)ₙ-R₂, wherein R₂ represents a nitrogen containing basic residue such as NH₂, NHR₄ or NR₅R₆, wherein R₄, R₅ and R₆ independently are C₁-C₄ alkyl or cycloalkyl and n is an integer of from 1 to 4 and R₃ represents hydrogen, C₁-C₄ alkyl/cycloalkyl group having not more than 4 carbon atoms, and the physiologically acceptable addition products of the compounds with acids and halogen adducts, preferably adducts with iodine, iodine monochloride or iodine mono-bromide,
for preparing a drug for treatment of multiple sclerosis.

2. Use according to claim 1 **characterized in that** it is a compound wherein R₁ is methyl in positions 2 and 3 and hydrogen in the other positions.

3. Use according to claim 1 or 2 **characterized in that** it is a compound wherein R₁ in one of the positions 7-10 is hydroxy; preferably in position 9.

4. Use according to any of the preceding claims **characterized in that** it is a compound wherein X is CH₂N(CH₃)₂ and R₃ is hydrogen.

## Patentansprüche

1. Verwendung einer Verbindung, die ein substituiertes Indolchinoxalin der allgemeinen Formel (I) ist: 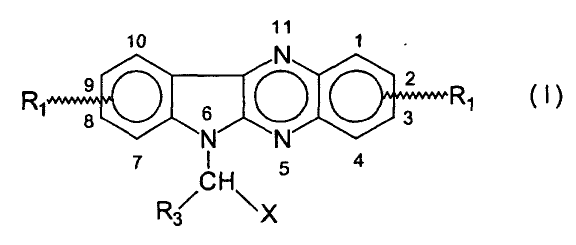 wobei R₁ Wasserstoff oder eine oder mehrere, bevorzugt 1 bis 4, ähnliche oder verschiedene Substituenten an den Positionen 1 bis 4 und/oder 7 bis 10 darstellt, die ausgewählt werden aus Halogen, vorzugsweise Br, einer Niederalkyl/Niederalkoxygruppe mit nicht mehr als 4 Kohlenstoffatomen, einer Trifluormethylgruppe, einer Trichlormethylgruppe; und an einer der Positionen 7 bis 10 kann R₁ eine Hydroxylgruppe sein;
X ist eine -(CH₂)ₙ-R₂-Gruppe, wobei R₂ einen stickstoffhaltigen basischen Rest, wie beispielsweise NH₂, NHR₄ oder NR₅R₆, darstellt, wobei R₄, R₅ und R₆ unabhängig voneinander C₁₋₄-Alkyl oder Cycloalkyl sind, und n eine ganze Zahl von 1 bis 4 ist, und R₃ Wasserstoff, eine C₁₋₄-Alkyl/Cycloalkylgruppe mit nicht mehr als 4 Kohlenstoffatomen darstellt, sowie die physiologisch annehmbaren Additionsprodukte dieser Verbindungen mit Säuren und Halogen-Addukten, bevorzugt Addukte mit Iod, Iodmonochlorid oder Iodmonobromid,
zur Herstellung eines Medikaments zur Behandlung von Multipler Sklerose.

2. Verwendung gemäss Anspruch 1, **dadurch gekennzeichnet, dass** es eine Verbindung ist, wobei R₁ an den Positionen 2 und 3 Methyl und an den anderen Positionen Wasserstoff ist.

3. Verwendung gemäss Anspruch 1 oder 2, **dadurch gekennzeichnet, dass** es eine Verbindung ist, wobei R₁ an einer der Positionen 7 bis 10, bevorzugt an der Position 9, Hydroxy ist.

4. Verwendung gemäss einem der vorhergehenden Ansprüche, **dadurch gekennzeichnet, dass** es eine Verbindung ist, in der X CH₂N(CH₃)₂ ist und R₃ Wasserstoff ist.

## Revendications

1. Utilisation d'un composé qui est une indoloquinoxaline substituée de formule générale I : 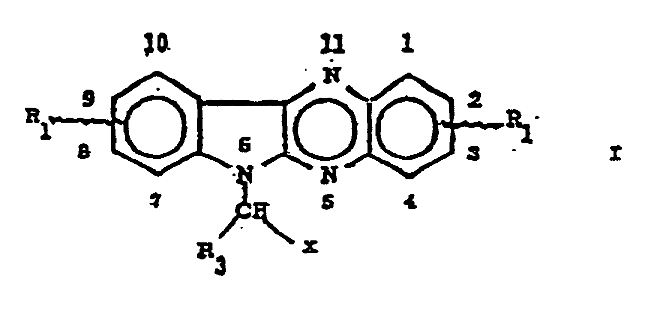 dans laquelle R₁ représente l'hydrogène ou un ou plusieurs, de préférence 1 à 4, substituants identiques ou différents en positions 1 à 4 et/ou 7 à 10, choisis parmi les halogène, de préférence brome, les groupes alkyle inférieur/alcoxy inférieur ne comportant pas plus de 4 atomes de carbone, groupe trifluorométhyle, groupe trichlorométhyle, et en l'une des positions 7 à 10, R₁ peut être un groupe hydroxyle ;
X est un groupe -(CH₂)ₙ-R₂, dans lequel R₂ représente un résidu basique contenant de l'azote tel que NH₂, NHR₄ ou NR₅R₆, où R₄, R₅ et R₆ sont indépendamment des alkyles en C₁ à C₄ ou cycloalkyles, et n est un entier de 1 à 4, et R₃ représente un hydrogène, un groupe alkyle en C₁ à C₄/cycloalkyle ne comportant pas plus de 5 atomes de carbone, et les produits d'addition physiologiquement acceptables des composés avec des acides et des aduits d'halogène, de préférence des aduits avec l'iode, le monochlorure d'iode ou le monobromure d'iode,
pour préparer un médicament destiné au traitement de la sclérose multiple.

2. Utilisation selon la revendication 1, **caractérisé en ce qu'**il s'agit d'un composé dans lequel R₁ est un méthyle en positions 2 et 3 et un hydrogène aux autres positions.

3. Utilisation selon l'une quelconque des revendications 1 ou 2, **caractérisé en ce qu'**il s'agit d'un composé dans lequel R₁ en l'une des positions 7 à 10 est un hydroxy, de préférence en position 9.

4. Utilisation selon l'une quelconque des revendications 1 à 3, **caractérisé en ce qu'**il s'agit d'un composé dans lequel X est CH₂N(CH₃)₂ et R₃ est l'hydrogène.
